# EUROPÄISCHE PATENTSCHRIFT

(11) **EP 1 057 462 B1**
(45) Veröffentlichungstag und Bekanntmachung des Hinweises auf die Patenterteilung: **02.04.2003**
(21) Anmeldenummer: 99110053.8
(22) Anmeldetag: 21.05.1999
(51) Int. Cl.: A61F 2/30, A61F 2/44

(54) **Zwischenwirbel-Endoprothese mit einer gezahnten Anschlussplatte**
Intervertebral endoprosthesis with a toothed connection plate
Endoprothèse intervertébrale avec une plaque de connexion dentée

(43) Veröffentlichungstag der Anmeldung: 06.12.2000
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 357 547
- EP-A- 0 522 999
- EP-A- 0 560 141
- FR-A- 2 641 461
- US-A- 3 840 904
- US-A- 5 702 450

## Beschreibung

Es ist eine Zwischenwirbel-Endoprothese bekannt (EP-B-176 728), die aus zwei Anschlußplatten besteht, die zwischen sich einen Gelenkkern einschließen. Die Anschlußplatten sind bestimmt zur Anlage an den Deckplatten der beiderseitigen Wirbelkörper. Damit die Prothese nicht unter Schubkräften, die hauptsächlich in anterior-posteriorer Richtung parallel zur Haupterstreckung der Anschlußplatten verlaufen, ihre vorbestimmte Lage zwischen den Wirbelkörpern verläßt, müssen die Anschlußplatten mit den Wirbelkörpern in geeigneter Weise verbunden werden. Zu diesem Zweck sind bei der bekannten Prothese randständige Zähne vorgesehen, die quer von jeder Anschlußplatte abragen, um unter der zwischen den Wirbelkörpern wirkenden, natürlichen Druckkraft in deren Deckplatten einzudringen. In diesem Zustand setzen sie einer Verschiebung der Anschlußplatten im Verhältnis zu den Deckplatten der Wirbelkörper in irgendeiner Richtung der Flächenerstreckung der Anschlußplatten und Wirbelkörper Widerstand entgegen. Damit dieser Widerstand verläßlich groß ist, sind die Zähne in der praktischen, durch Benutzung bekannt gewordenen Ausführung der genannten Endoprothese quer zur Richtung der Flächenerstreckung der Anschlußplatten bzw. Deckplatten flächig ausgebildet. Sie sind nämlich von einem Paar im wesentlichen dreieckiger Hauptflächen begrenzt, die längs ihrer im wesentlichen parallelen Basisseiten mit der Anschlußplatte verbunden sind und deren der Basisseite gegenüberliegende, freie Spitze von der Anschlußplatte wegweist. Damit die Zähne hinreichende Stabilität aufweisen, sind sie mit einer gewissen Dicke ausgeführt, die dem Abstand der parallel zueinander liegenden Dreiecksflächen entspricht. Die Kanten der Dreiecksflächen, die sich zwischen ihrer Basisseite und ihrer Spitze erstrekken, sind jeweils durch rechteckige, ebene Flächen miteinander verbunden, die zur Zahnspitze hin zusammenlaufen und dort einen First bilden.

Bei der Verwendung der Prothese hat sich herausgestellt, daß die Zähne in manchen Fällen nicht hinreichend tief in die Deckplatten der Wirbelkörper eindringen. Es besteht dann die Gefahr, daß sie ihre Aufgabe nicht sicher erfüllen können, die Prothese in der vorgesehenen Position zu halten. Erfahrungsgemäß muß man in derartigen Fällen Zuflucht nehmen zu der Verwendung von Prothesenzapfen, die in vorgebohrte Löcher der Wirbelkörper eingesetzt werden (DE-C-30 23 353, FR-A-2 659 226). Jedoch ist eine solche Bearbeitung der Wirbelkörper schwierig. Sie verlängert die Operationsdauer und schwächt die Wirbelkörper. Die Verwendung spitzer, pyramidenförmiger Zähne verbietet sich deshalb, weil die Zähne im Hinblick auf die Zahnstabilität zum Fuß hin dicker werden und demzufolge von Schrägflächen begrenzt werden, die unter Schubkräften dazu führt, die Zähne vom Knochen abzuheben.

Die der Erfindung zugrunde liegende Aufgabe besteht darin, die eingangs erwähnte Zwischenwirbel-Endoprothese derart weiterzubilden, daß die Zähne hinreichend sicher in die Deckplatte des jeweils zugeordneten Wirbelkörpers eindringen und darin auch gegen die Wirkung von Schubkräften sicher verankert sind. Die erfindungsgemäße Lösung besteht in den Merkmalen des Anspruchs 1 und vorzugsweise denen der Unteransprüche.

Demgemäß ist eine Zwischenwirbel-Endoprothese vorgesehen, die eine in Anlage an einer Wirbeloberfläche zu fixierende Anschlußplatte aufweist. Diese ist in einer quer zu ihrer Haupterstreckung verlaufenden Ansetzrichtung an die Wirbeloberfläche anzusetzen. Sie weist zwecks Schubsicherung gegenüber einer parallel zu ihrer Haupterstreckung verlaufenden Schubkraft etwa in der Ansetzrichtung vorragende Zähne auf. Diese sind u. a. von einer Hauptfläche begrenzt, die in der Ansetzrichtung und quer zur Schubrichtung angeordnet ist. Anders als bei üblicher, pyramidenförmiger Zahngestalt, bei welcher die den Zahn begrenzenden Flächen unter gleichem Winkel gegenüber der Ansetzrichtung geneigt sind, sind die erfindungsgemäßen Zähne im Verhältnis zur Ansetzrichtung schief ausgebildet, indem nämlich die Hauptfläche parallel zur Ansetzrichtung verläuft und die Verbreiterung des Zahns von der Spitze zu seiner Grundfläche hin, die für seine Stabilität erforderlich ist, allein durch Schrägung der übrigen Flächen erbracht wird.

Zwar ist eine nicht gattungsgemäße Hüftgelenk-Endoprothese bekannt (FR 2 641 461 A1), deren im Hüftgelenkknochen zu verankernder halbkugelförmiger Teil am Außenrand einen Kranz von Keilen vorsieht, die in Einpaßrichtung spitz zulaufen. Die Außenflächen der Keilkörper liegen auf einer zylindrischen Fläche, deren Zylinderachse in Ansetzrichtung verläuft. Es bleibt jedoch im dunkeln, ob und ggf. welcher technische Effekt mit der zylindrischen Gestaltung der äußeren Keilflächen bewirkt wird.

Ferner zeichnet sich die Erfindung dadurch aus, daß je zwei Zähne oder Zahngruppen einander in der Schubrichtung gegensinnig gegenüberstehen. Das bedeutet, daß die Hauptflächen von je zwei paarweise zusammengehörigen Zähnen entweder einander zugewendet oder voneinander abgewendet sind. Der Sinn dieser Maßnahme liegt darin, daß die quer zur Ansetzrichtung verlaufenden Kräfte, die beim Eindringen der Schrägflächen in dem Wirbel erzeugt werden, entgegengesetzt gerichtet sind und sich daher aufheben. Die Zähne können daher in der vorgesehenen Ansetzrichtung in den Wirbel eindringen, d. h. in der Richtung der Hauptflächen.

Die erfindungsgemäße Formgebung der Zähne hat zwei wesentliche Vorteile. Der eine besteht darin, daß beim Auftreten einer Schubkraft parallel zur Haupterstreckung der Anschlußplatte stets mindestens eine Zahn-Hauptfläche vorhanden ist, die lotrecht zur Richtung dieser Schubkraft verläuft und daher die besten Voraussetzungen für eine Lagesicherung des Implantats gegenüber dieser Schubkraft bietet. Die bei pyramidenförmigen Zähnen bestehende Gefahr, daß das Implantat durch Bewegung in der Schubrichtung entlang einer schrägen Zahnfläche aus der Anlageposition am Wirbel herausgehoben wird, wird vermieden.

Der zweite Vorteil besteht darin, daß die Wirbelflächen, mit denen die Hauptflächen der Zähne beim Eindringen zusammenwirken und an denen sie sich gegenüber Schubkräften abstützen werden, geschont werden. Schräg zur Ansetzrichtung verlaufende Zahnflächen führen beim Eindringen in die Wirbelrinde dazu, daß diese fortschreitend ausbricht, um den wachsenden Querschnitt des eindringenden Zahns den Platz zu räumen. Dieses vielfache Ausbrechen beeinträchtigt die Stabilität des davon betroffenen Teils des Wirbels. Im Gegensatz dazu findet an den in der Ansetzrichtung verlaufenden Hauptflächen des Zahns kein fortschreitendes Ausbrechen statt. Die betreffenden Teile des Wirbels werden daher geschont, bleiben stabiler und stützen den Zahn besser ab. Die Integrität und Kraftaufnahmefähigkeit der Wirbelrinde der Wirbel-Deckplatten bleibt im Bereich der eindringenden Zähne besser erhalten und deshalb kann in der Regel auf zusätzliche Befestigungsmittel wie Schrauben verzichtet werden.

Das gilt insbesondere dann, wenn die Kanten der Hauptfläche scharf ausgebildet sind, so daß sie während des Eindringens in den Knochen diesen mehr schneiden als wegbrechen. Wenn die Hauptfläche eben ist, kann man die Schärfe der Hauptflächenkanten dadurch herbeiführen, daß man die an die Hauptfläche angrenzenden Flächen des Zahns unter einem spitzen Winkel (in einem Querschnitt parallel zur Haupterstreckung der Anschlußplatte) anordnet. Die Grundfläche des Zahns erlangt dadurch beispielsweise eine dreiecksförmige oder trapezförmige Gestalt. Es ist jedoch nicht unbedingt erforderlich, daß die Hauptfläche eben ist; sie kann sich vielmehr auch aus zwei oder mehr in vorzugsweise stumpfen Winkel aneinander anschließenden ebenen Flächen zusammensetzen oder mehr oder weniger gleichmäßig gekrümmt ausgebildet sein.

Die Ansetzrichtung ist diejenige Richtung, unter der man normalerweise das Implantat an den Wirbel ansetzen möchte. Wenn die Anschlußplatte eben ist, steht sie normalerweise lotrecht zur Plattenebene. Wenn die Anschlußplatte gekrümmt ist, ergibt sich die Ansetzrichtung im allgemeinen natürlicherweise als Lotrechte auf die durchschnittliche Erstrekkungsrichtung der Anschlußplatte. Im übrigen ist sie am Produkt erkennbar als diejenige Richtung, unter der die Hauptflächen parallel zueinander verlaufen.

Die Schubkräfte, gegenüber denen die Zähne die Zwischenwirbel-Endoprothese am Wirbel abstützen sollen, treten in der Regel als Komponenten von irregulär gerichteten Kräften auf, wobei in anterior-posteriorer Richtung besonders hohe und für die Fixierung des Implantats am Wirbel besonders bedrohliche Kraftkomponenten verlaufen. Daher ist es besonders wichtig, die Anschlußplatten in anterior-posteriorer Richtung gegenüber den Deckplatten der Wirbelkörper zu fixieren. Die Hauptflächen der an den Anschlußplatten des Implantats vorgesehenen Zähne sind daher in AP-Richtung und in benachbarten Richtungen orientiert. Es empfiehlt sich, zwei Zahngruppen vorzusehen, von denen die eine gegenüber den nach anterior gerichteten Kräften und die andere gegenüber den nach posterior gerichteten Kräften abstützt. Diese Gruppen werden derart gegensinnig angeordnet, daß die durch ihre Schrägflächen beim Eindringen in den Knochen erzeugten, quer zur Ansetzrichtung verlaufenden Kräfte einander im wesentlichen aufheben.

Dafür kann insbesondere eine symmetrische Anordnung in bezug auf eine quer zur Richtung der Schubkraft verlaufenden Symmetrieachse oder in bezug auf einen sternsymmetrischen Mittelpunkt geeignet sein; erforderlich ist Symmetrie aber nicht.

Weitere Merkmale der Erfindung ergeben sich aus der folgenden Beschreibung eines Ausführungsbeispiels, das in der Zeichnung dargestellt ist. Es zeigen:
- Fig. 1: einen von dorsal oder ventral gesehenen Schnitt durch eine Zwischenwirbel-Endoprothese zwischen zwei teilweise dargestellten Wirbelkörpern;
- Fig. 2: eine Draufsicht auf die bezahnte Seite einer Anschlußplatte;
- Fig. 3: einen Schnitt längs der Linie III-III der Fig. 2; und
- Fig. 4, 5 und 6: eine Draufsicht und zwei Seitenansichten eines Zahns in vergrößerter Darstellung.

Die Zwischenwirbel-Endoprothese 1, die zwischen zwei Wirbelkörper 2 einzusetzen ist, besteht aus zwei Anschlußplatten 3 und einem Gelenkkern 4. Die Außenseiten 5 der Anschlußplatten 3 tragen mehrere spitze Zähne 6, die durch eine in Pfeilrichtung 7 wirkende Kraft, zu der auch die zwischen den Wirbelkörpern 2 wirkende natürliche Druckkraft gehören kann, in deren kortikale Endplatten 8 eingetrieben werden, um die gewünschte Position der Prothese zu sichern.

Die Zähne 6 sind in zwei Gruppen jeweils nahe den ventralen bzw. dorsalen Rändern 9 der Anschlußplatte 3 angeordnet, und zwar symmetrisch zu einer in Ventral-Dorsal-Richtung verlaufenden Diametralachse 10. Jeweils der mittlere Zahn jeder Gruppe ist auf diese Diametralrichtung 10 ausgerichtet, während die weiter außen liegenden Zähne einen Winkel α mit der Diametralrichtung einschließen, der in der Größenordnung von 30° liegt und größer als 20° sein soll. Der Grund dafür liegt darin, daß die Schubkräfte, gegenüber denen die Zähne die Prothese an den Wirbelkörpern festhalten sollen, hauptsächlich, aber nicht nur, in AP-Richtung verlaufen.

Jeder Zahn wird von vier Flächen begrenzt, nämlich von einer Hauptfläche 11, zwei Seitenflächen 13 und einer Rückfläche 12. Die Hauptfläche 11 verläuft unter einem Winkel von 90° zur Anschlußplatte 3, genauer gesagt zu derjenigen Ebene, die die durchschnittliche Erstreckungsrichtung der die Zähne tragenden Außenfläche der Anschlußplatte 3 angenähert wiedergibt. Im Ausführungsbeispiel sind die lotrecht zur Anschlußplatte 3 verlaufenden Hauptflächen 11 nach außen (von der Mitte der Diametralachse 10 weg) gerichtet. Es könnte auch umgekehrt sein.

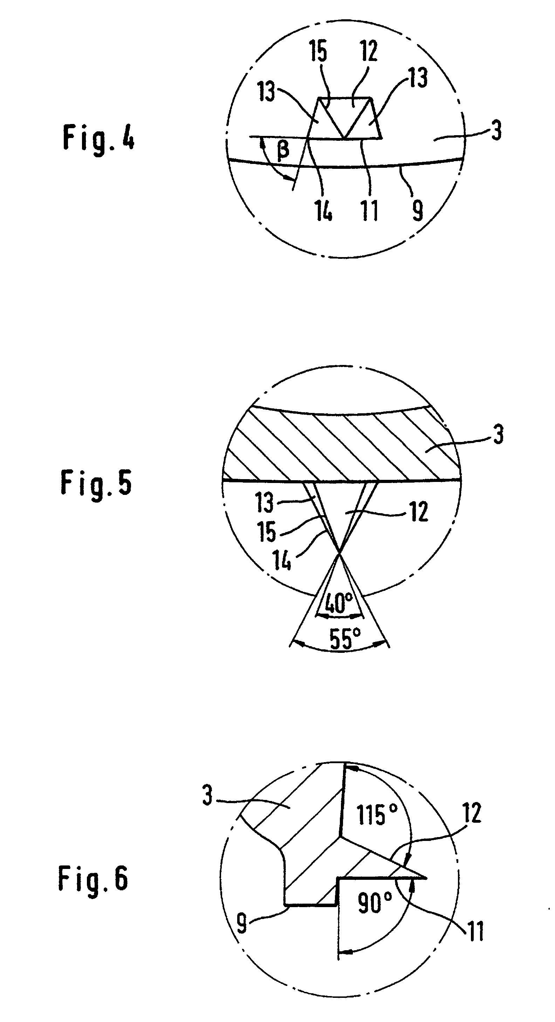

Die gegenüberliegende Rückfläche 12 verläuft in einem parallel zur Anschlußplatte verlaufenden Schnitt durch den Zahn parallel zur Hauptfläche 11. Im Vertikalschnitt (Fig. 6) verläuft sie schräg gegenüber der Anschlußplatte 3, nämlich im dargestellten Beispiel unter einem Winkel von 115°. Dieser Winkel liegt vorzugsweise zwischen 105 und 145°.

Die Kanten 14, 15 der Hauptfläche 11 und der Rückfläche 12 werden durch die Seitenflächen 13 verbunden, die gleichfalls dreieckig sind und zu derselben Spitze konvergieren wie die Haupt- und Rückflächen 11, 12. Im Verhältnis zur Hauptfläche 11 verlaufen sie in einem parallel zur Anschlußplatte 3 verlaufenden Schnitt unter einem spitzen Winkel β, der kleiner sein soll als 80°. Die Kante 14 zwischen der Hauptfläche 11 und den Seitenflächen 13 wird dadurch zu einer Art Schneide.

Der gegenüber der Hauptfläche 11 spitzwinklige Verlauf der Seitenflächen 13 kommt dadurch zustande, daß der Spitzenwinkel der Hauptfläche 11 ein wenig größer ist als derjenige der Rückfläche 12. In Fig. 5 wurden diese Winkel mit 55 bzw. 40° angegeben. Je größer der Unterschied zwischen diesen beiden Winkeln ist, um so schärfer ist die von der Kante 14 gebildete Schneide. Der Spitzenwinkel der Hauptfläche 11 soll kleiner als 70° vorzugsweise kleiner als 60° sein, während der Spitzenwinkel der Rückfläche um 10 bis 25°, vorzugsweise etwa 15° geringer ist.

Dies wirkt sich beim Eindrücken der Zähne in die Knochenrinde der Wirbelkörper folgendermaßen aus. Die Hauptflächen 11 verlaufen parallel zur Eindringbewegung. Sobald die Spitze eines Zahns die Knochenrinde durchstoßen hat, können die Hauptflächen 11 daher entlang der anfänglich gebildeten Schnitt- oder Eindringkante weiter voranschreiten. Die Länge dieser Kante vergrößert sich bei fortschreitendem Eindringen entsprechend der zunehmenden Breite der Hauptfläche 11. Da diese Verbreiterung im Wirkungsbereich der schneidenförmigen Kante 14 stattfindet, findet sie mehr unter Schnitt- als unter Bruchwirkung statt. Die mit der Hauptfläche 11 schließlich zusammenwirkende Knochenkante ist daher weitgehend frei von Bruchschädigung und daher besser in der Lage, die von der Hauptfläche 11 übertragenen Kräfte aufzunehmen.

Hingegen findet das Eindringen der Rückfläche 12 sowie der Seitenflächen 13 unter fortschreitender Verdrängung der damit jeweils zusammenwirkenden Bruchkanten der Knochenrinde statt. Dies ist jedoch nicht von Nachteil, da die Abstützung des Implantats gegenüber Schubkräften hauptsächlich von der Hauptfläche 11 übernommen wird.

Damit eine gute Kraftübertragung in allen Hauptrichtungen stattfinden kann, sind die Hauptflächen 11 von einander diametral gegenüberliegend angeordneten Zähnen gegensinnig gerichtet.

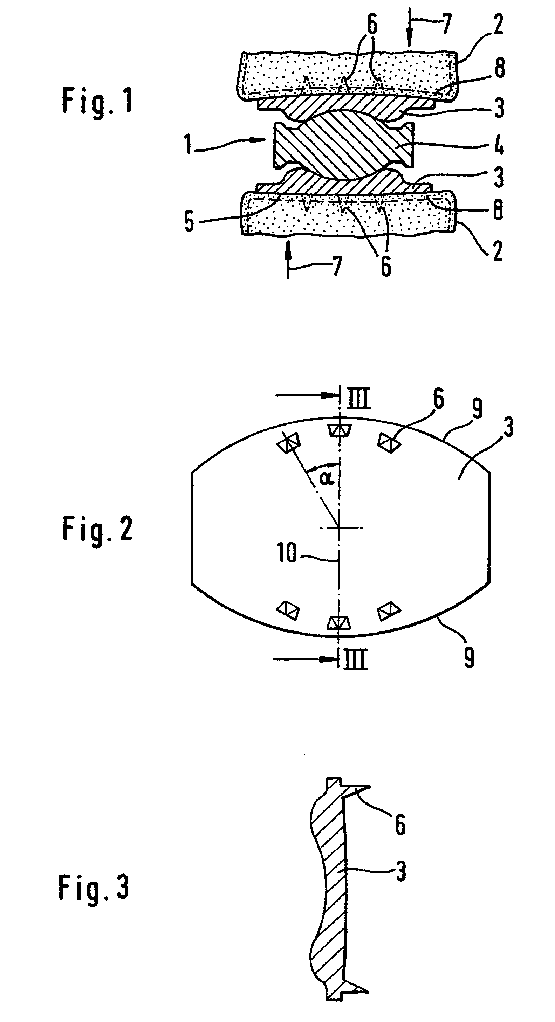

Es hat sich gezeigt, daß die erfindungsgemäß gestalteten Zähne größere Sicherheit für die Positionshaltung der Prothese geben als die bisher bekannten, wenn eine etwa gleiche Zahnflächengröße quer zur AP-Richtung 10 (Fig. 2) zugrunde gelegt wird.

## Patentansprüche

1. Zwischenwirbel-Endoprothese mit einer in Anlage an einer Wirbeloberfläche zu fixierenden Anschlußplatte (3), die in einer quer zu ihrer Haupterstreckung verlaufenden Ansetzrichtung an die Wirbeloberfläche anzusetzen ist und zur Schubsicherung gegenüber einer parallel zu ihrer Haupterstreckung verlaufenden Schubkraft etwa in der Ansetzrichtung vorragende Zähne trägt, die von Flächen (11, 12, 13) begrenzt sind, **dadurch gekennzeichnet, daß** diese Flächen eine Hauptfläche (11) umfassen, die in der Ansetzrichtung und quer zur Schubrichtung angeordnet ist, wobei je zwei Zähne (6) oder Zahngruppen in der Schubrichtung einander gegensinnig gegenüberstehen.

2. Zwischenwirbel-Endoprothese nach Anspruch 1, **dadurch gekennzeichnet, daß** die Hauptflächen (11) von einander gegensinnig gegenüberstehenden Zähnen (3) voneinander weg gerichtet sind.

3. Zwischenwirbel-Endoprothese nach Anspruch 1 oder 2, **dadurch gekennzeichnet, daß** die Kanten (14) der Hauptflächen (11) scharf ausgebildet sind.

4. Zwischenwirbel-Endoprothese nach Anspruch 3, **dadurch gekennzeichnet, daß** in einem parallel zur Anschlußplatte (3) verlaufenden Querschnitt durch die Zähne (6) deren Seitenflächen (13) mit der Hauptfläche (11) einen Winkel (β) von nicht mehr als 80° einschließen.

5. Zwischenwirbel-Endoprothese nach Anspruch 4, **dadurch gekennzeichnet, daß** die Grundfläche der Zähne (6) trapezförmig ist, wobei die größte Seite der Hauptfläche (11) zugeordnet ist.

6. Zwischenwirbel-Endoprothese nach einem der Ansprüche 1 bis 5, **dadurch gekennzeichnet, daß** je eine Gruppe von Zähnen (6) in zwei gegenüberliegenden Randbereichen der Anschlußplatte (3) vorgesehen ist.

7. Zwischenwirbel-Endoprothese nach Anspruch 5, **dadurch gekennzeichnet, daß** die Hauptflächen (11) der Zähne (6) jeder Gruppe in einem Winkel (α) von mindestens ± 20° gegenüber der Diametralrichtung (10) unterschiedlich orientiert sind.

## Claims

1. An intervertebral endoprosthesis, with a connection plate (3) which is to be fixed on a vertebral surface and which is to be applied to the vertebral surface in a direction of application running transverse to its main extension, and, in order to protect against a shearing force running parallel to its main extension, the connection plate (3) has teeth projecting approximately in the direction of application, which teeth are delimited by surfaces (11, 12, 13) **characterized by** that said teeth include a main surface (11) arranged in the direction of application and transverse to the direction of shearing, two teeth (6) or groups of teeth in each case lying opposite one another in the direction of shearing and pointing in opposite directions.

2. The intervertebral endoprosthesis as claimed in claim 1, wherein the main surfaces (11) of teeth (6) lying opposite one another and pointing in opposite directions are oriented so as to face away from one another.

3. The intervertebral endoprosthesis as claimed in claim 1 or 2, wherein the edges (14) of the main surfaces (11) are sharp.

4. The intervertebral endoprosthesis as claimed in claim 3, wherein, in a cross section parallel to the connection plate (3), the side surfaces (13) of the teeth (6) enclose an angle (β) of not more than 80° with the main surface (11).

5. The intervertebral endoprosthesis as claimed in claim 4, wherein the main surface of the teeth (6) is trapezoidal, the greatest side being assigned to the main surface (11).

6. The intervertebral endoprosthesis as claimed in one of Claims 1 through 5, wherein a group of teeth (6) is in each case provided in two opposite edge areas of the connection plate (3).

7. The intervertebral endoprosthesis as claimed in claim 5, wherein the main surfaces (11) of the teeth (6) of each group are oriented differently at an angle (α) of at least ± 20° in relation to the diametral direction (10).

## Revendications

1. Endoprothèse intervertébrale avec une plaque de connexion (3) à fixer en contact avec une surface d'une vertèbre, qui doit être appliquée sur la surface de la vertèbre dans une direction d'application dirigée transversalement par rapport à son étendue principale et qui porte des dents, limitées par des surfaces (11, 12, 13), qui saillent à peu près dans la direction d'application pour l'assurer en cisaillement par rapport à une force de cisaillement dirigée parallèlement à son étendue principale, **caractérisée en ce que** ces surfaces comprennent une surface principale (11), qui est disposée dans la direction d'application et transversalement par rapport à la direction de cisaillement, deux dents (6) ou groupes de dents symétriques se faisant face respectivement dans la direction du cisaillement.

2. Endoprothèse intervertébrale selon la revendication 1, **caractérisée en ce que** les surfaces principales (11) de dents (3) orientées en sens inverse et diamétralement opposées sont détournées l'une de l'autre.

3. Endoprothèse intervertébrale selon la revendication 1, **caractérisée en ce que** les arêtes (14) des surfaces principales (11) sont configurées à angles vifs.

4. Endoprothèse intervertébrale selon la revendication 3, **caractérisée en ce que**, dans une section à travers les dents (6) s'étendant parallèlement à la plaque de connexion (3), les surfaces latérales (13) de ces dernières forment avec la surface principale (11) un angle (β) de pas plus de 80°.

5. Endoprothèse intervertébrale selon la revendication 4, **caractérisée en ce que** la surface de base des dents (6) est trapézoïdale, le grand côté étant affecté à la surface principale (11).

6. Endoprothèse intervertébrale selon une des revendications 1 à 5, **caractérisée en ce qu'**un groupe de dents (6) est prévu dans chacune de deux zones de bordure opposées de la plaque de connexion (3).

7. Endoprothèse intervertébrale selon la revendication 5, **caractérisée en ce que** les surfaces principales (11) des dents (6) de chaque groupe sont orientées différemment selon un angle (α) d'au moins ± 20° par rapport à la direction du diamètre (10).
